# EUROPEAN PATENT APPLICATION

(11) **EP 4 473 903 A2**
(43) Date of publication of application: **11.12.2024**
(21) Application number: 24174371.5
(22) Date of filing: 06.05.2024
(51) Int. Cl.: A61B 5/055

(54) **MAGNETIC RESONANCE IMAGING APPARATUS, BODY MOVEMENT INFORMATION PRESENTATION METHOD, AND MEDICAL IMAGE DIAGNOSIS APPARATUS**

(30) Priority: 18.05.2023 JP 2023082589
(71) Applicant: FUJIFILM Corporation, Tokyo 106-8620 (JP)
(72) Inventor: YOKOHAMA, Wataru, Chiba, 277-0804 (JP)
(74) Representative: Strehl Schübel-Hopf & Partner

(57) **Abstract**

Provided is a technique that enables a subject to be aware of his/her own body movement in relation to imaging in a long-time MRI examination, and thus enables the subject to participate in the examination in a cooperative manner without feeling bored or anxious.

An MRI apparatus includes a body movement information processing unit that uses body movement information from a body movement detection device that detects body movement of a subject disposed in an imaging space and imaging information of the imaging unit to display body movement-related information, in which the body movement information of the subject and the imaging information are associated with each other, on a video display device disposed at a position where the subject disposed in the imaging space is capable of visually recognizing the body movement-related information.

## Description

### INCORPORATION BY REFERENCE

The present application claims priority from Japanese patent application JP-2023-082589 filed on May 18, 2023, the content of which is hereby incorporated by reference into this application.

### BACKGROUND OF THE INVENTION

### 1. Field of the Invention

The present invention relates to a medical image diagnosis apparatus that inserts a subject into a predetermined bore and images the subject, typified by a magnetic resonance imaging apparatus (hereinafter, referred to as an MRI apparatus) or a CT apparatus, and particularly relates to a technique for providing the subject placed in the bore with information.

### 2. Description of the Related Art

An MRI examination is performed in a cylindrical imaging space called a "bore" of an MRI apparatus, and an examination time is relatively long, 20 to 30 minutes. In a case where the subject moves during the examination, a body movement artifact occurs in an image, and an accurate image cannot be obtained. Therefore, it is necessary to suppress body movement including respiration as much as possible during the examination. However, there are not a few patients who feel pain or stress by being in a closed space called a bore for a long time while suppressing body movement to receive an examination. In addition, some patients may get bored and fall asleep during the examination, and progress of the examination may be delayed. Such a situation is often seen in elderly people or middle-aged and older people who are fatigued in daily life, in examinations such as abdominal and cardiac examinations that tend to take a long time. Furthermore, during a long examination, many patients feel anxious about what level of body movement has no influence on the examination. An examination using a CT apparatus is usually performed in a shorter time than the MRI examination, but in a case where a contrast medium is used, it may take about 5 minutes to 20 minutes, resulting in a similar problem.

Various techniques, such as a technique for suppressing movement of a subject during imaging as much as possible, a method of detecting body movement and determining whether or not reimaging is required based on the detected body movement, and an image processing technique of correcting an image by using information on body movement, have been proposed (JP2006-346235A). Since these are handled on a side that performs the imaging, such as side of a technician, a doctor, or an apparatus, information related to the body movement is not fed back to the subject.

On the other hand, efforts have been made to display a video of a landscape, an animal, or the like at a position that can be seen by a subject so that the subject does not fall asleep or get bored because of a long imaging time.

### SUMMARY OF THE INVENTION

Although a problem of image quality degradation caused by body movement during an examination can be solved to some extent by an image processing technique of correcting the body movement, reimaging is required depending on a magnitude of the body movement, and a restraint time of the subject is further increased. However, since the handling that is processed on the apparatus side or the operator side is not fed back to the subject, the subject cannot be relieved of anxiety.

On the other hand, the display of a landscape, an animal, or the like is an effort to prevent the subject being examined from getting bored, but cannot directly solve the problem of the body movement in which the subject moves during an examination.

An object of aspects of the present invention is to propose a technique that enables a subject to be aware of his/her own body movement in relation to imaging in a long-time MRI examination, and thus enables the subject to participate in the examination in a cooperative manner without feeling bored or anxious.

Aspects of the present invention provide a medical image diagnosis apparatus comprising units that detect body movement of a subject and present, to the subject, information on the detected body movement in association with currently performed imaging, particularly an MRI apparatus.

That is, a medical image diagnosis apparatus according to an aspect of the present invention comprises an imaging unit and a body movement information processing unit. The body movement information processing unit displays body movement-related information, in which body movement information from a body movement detection device that detects the body movement of the subject disposed in an imaging space and imaging information of the imaging unit are associated with each other, at a position where the subject disposed in the imaging space is capable of visually recognizing the body movement-related information, using a video display device.

In addition, an MRI apparatus according to an aspect of the present invention comprises an imaging unit configured to acquire a magnetic resonance signal of a subject and generate an image of the subject; and a body movement information processing unit configured to display body movement-related information, in which body movement information from a body movement detection device that detects body movement of the subject disposed in an imaging space and imaging information of the imaging unit are associated with each other, at a position where the subject disposed in the imaging space is capable of visually recognizing the body movement-related information, using a video display device.

In addition, a method of presenting, to a subject disposed in an imaging space and being examined, body movement information of the subject himself/herself is provided. The presenting is performed via a video display unit, and the presented body movement-related information includes a message such as a warning, a positive evaluation for the subject maintaining a stationary state, content reflecting the evaluation and entertaining the subject, and the like.

That is, in the method of an aspect of the present invention, a magnitude of body movement of the subject is analyzed for each part and whether or not the body movement is allowed is determined based on a relationship between an examination part and a part where the body movement is detected; and a warning is issued in a case where the body movement is not allowed. Alternatively, body movement of the subject and a duration time of a stationary state are monitored; and a video that changes in accordance with the duration time of the stationary state is displayed.

According to the present invention, since the imaging in progress and a state of the body movement of the subject himself/herself are presented to the subject being examined, the subject is motivated to consciously suppress the body movement, and it is easy to obtain consent even in a case where reimaging or the like due to the body movement occurs, so that it is possible to reduce discomfort caused by the restraint of the subject being examined. According to the present invention, the subject is required to be in the stationary state, and the subject can actively cooperate to maintain the stationary state instead of simply being passive to the imaging, and as a result, it is possible to reduce reimaging or the like and improve the image quality.

### BRIEF DESCRIPTION OF THE DRAWINGS

Fig. 1 is a diagram showing an outline of an MRI apparatus to which the present invention is applied.
Fig. 2 is a block diagram of an imaging unit of the MRI apparatus.
Fig. 3 is a functional block diagram of a calculation unit of an MRI apparatus according to an embodiment.
Fig. 4 is a diagram showing an outline of an operation of the MRI apparatus according to the embodiment.
Fig. 5 is a diagram showing processing of a body movement information processing unit according to Embodiment 1.
Figs. 6A and 6B are diagrams showing examples of body movement-related information displayed on a video display device according to Embodiment 1.
Fig. 7 is a diagram showing a modification example of the display according to Embodiment 1.
Fig. 8 is a diagram showing processing of a body movement information processing unit according to Embodiment 2.
Fig. 9 is a diagram showing an example of a table used by the body movement information processing unit according to Embodiment 2.
Fig. 10 is a diagram showing another example of the table used by the body movement information processing unit according to Embodiment 2.
Fig. 11 is a diagram showing an example of an examination flow used by a body movement information processing unit according to Embodiment 3.
Fig. 12 is a diagram showing an example of body movement-related information displayed on a video display device according to Embodiment 3.
Fig. 13 is a diagram showing processing of a body movement information processing unit according to Embodiment 4.
Fig. 14 is a diagram showing an example of body movement-related information (content) displayed on a video display device according to Embodiment 4.
Figs. 15A and 15B are diagrams showing other examples of the body movement-related information (content) displayed on the video display device according to Embodiment 4.
Figs. 16A and 16B are diagrams showing examples of body movement-related information displayed on a video display device according to Embodiment 5.

### DESCRIPTION OF THE PREFERRED EMBODIMENTS

Hereinafter, a medical image diagnosis apparatus according to the embodiments of the present invention will be described. In the following embodiments, an MRI apparatus will be described as an example, but the present invention can be applied to other image diagnosis apparatuses.

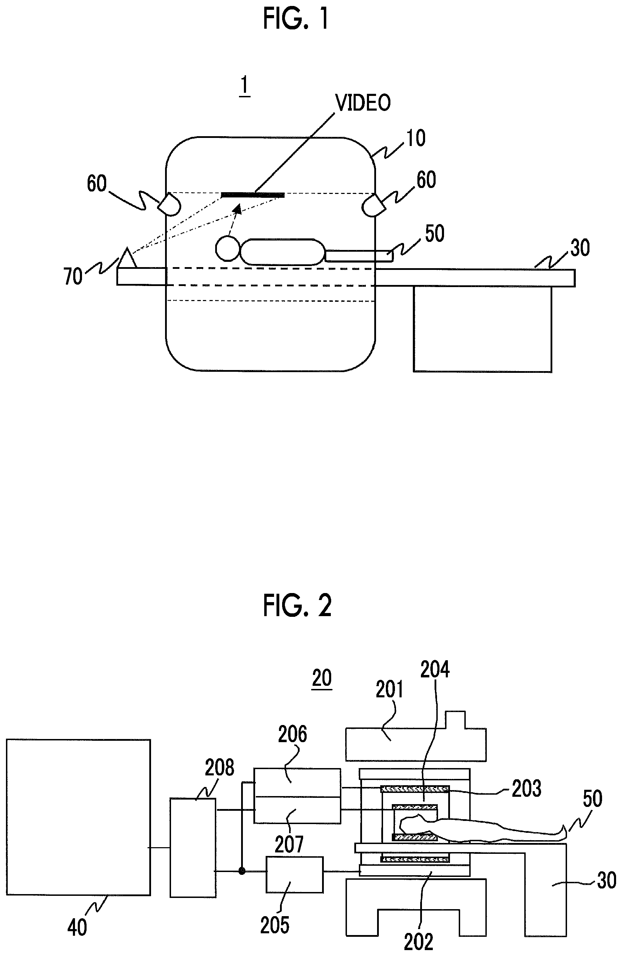

As shown in Fig. 1, an MRI apparatus 1 includes a gantry 10 that provides an imaging space (bore), and a table 30 on which a subject 50 is placed and that is inserted into the bore of the gantry 10, and an imaging unit (not shown) that applies a high-frequency magnetic field to the subject 50 and thereby collects a magnetic resonance signal is built in the gantry 10. Although not shown in Fig. 1, an operating part (console) and a calculation unit that controls the imaging unit and performs various calculations such as image reconstruction are provided separately from an examination room where the gantry 10 is placed.

The gantry 10 comprises a body movement detection device 60 that detects body movement of the subject 50 disposed in the imaging space, and a video display device 70 that displays a predetermined video at a position visible from the subject 50, monitors movement of the subject 50 during an MRI examination and feeds back information thereof as a video to the subject 50.

The body movement detection device 60 is not particularly limited as long as movement of a subject can be identified, but is configured with, for example, a camera such as an optical camera or an infrared camera, cameras (including a stereo camera), sensors such as an optical sensor, an infrared sensor, a microwave sensor, and a radio wave sensor. These body movement detection devices 60 are installed at one or a plurality of locations of the gantry 10 to monitor the movement of the subject. The body movement detection device 60 may include equipment that detects respiratory movement or a cardiac cycle, for example, an abdominal pressure gauge or an electrocardiograph.

The video display device 70 is a device that projects a video into a visual field of the subject inside the gantry, and a display, a liquid crystal display, an aerial display, or a projector can be used. In a case of a projector, a video may be directly projected into the bore or may be projected into the bore via a mirror.

The body movement detection device 60 and the video display device 70 are connected to a calculation unit (not shown) of the MRI apparatus. The body movement detection device 60 transmits the detected body movement information to the calculation unit. Further, the video display device 70 receives and displays body movement-related information generated by the calculation unit by using the body movement information.

A configuration of the imaging unit 20 is the same as that of a general MRI apparatus, and for example, as shown in Fig. 2, the imaging unit 20 comprises a static magnetic field magnet 201, gradient magnetic field coils 202 in three axis-directions and a power source 205 thereof, an RF transmission coil 203 and a transmitter 206, an RF reception coil 204 and a receiver 207, and the like, and further comprises a gradient magnetic field power source, and a sequencer 208 that operates a transmitter and a receiver in accordance with a predetermined pulse sequence.

The subject 50 is positioned, such that an examination part (a part to be examined) is positioned at a center of a static magnetic field space (imaging space) generated by the static magnetic field magnet 201, in the imaging space and imaging is performed. The imaging is performed by the RF reception coil 204 detecting a nuclear magnetic resonance signal generated from the subject 50 by irradiating the RF transmission coil 203, and in this case, positional information is added to the nuclear magnetic resonance signal by driving the gradient magnetic field coil 202 of each axis and applying a gradient magnetic field, and collection of a required number of nuclear magnetic resonance signals for image reconstruction is performed.

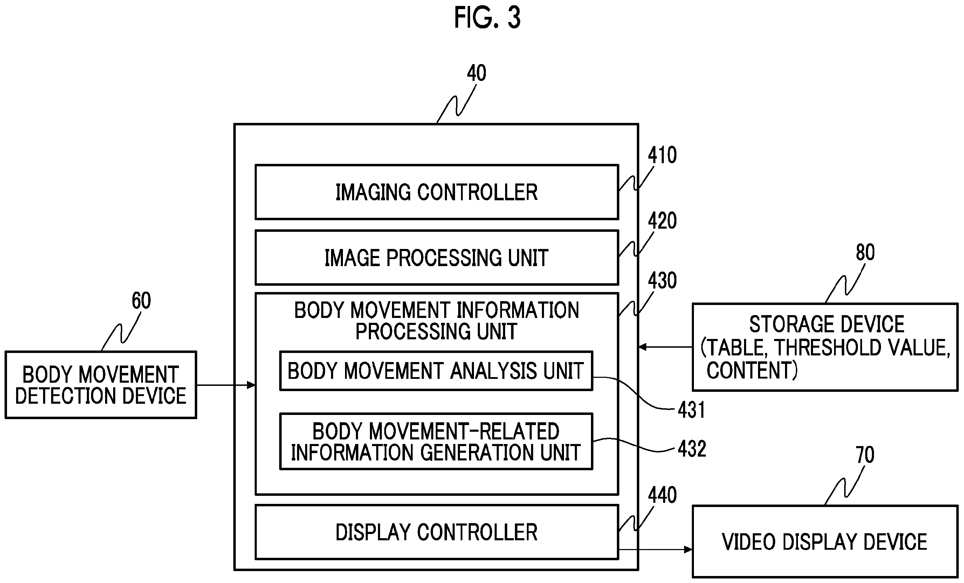

A calculation unit 40 performs control of the imaging unit 20 via the sequencer 208, the image reconstruction using the nuclear magnetic resonance signal (digital signal) collected by the receiver, image processing, and the like. Further, the calculation unit according to the present embodiment performs processing related to the body movement and processing for presenting a relationship between the body movement and the imaging to the subject being examined. Fig. 3 shows an example of a function of the calculation unit. In the example in Fig. 3, the calculation unit 40 comprises an imaging control unit 410, an image processing unit 420, a body movement information processing unit 430, and a display control unit 440. Functions of the imaging control unit 410 and the image processing unit 420 are the same as those of a general MRI apparatus, the imaging control unit 410 performs control of an examination flow and an imaging sequence via the sequencer 208, and the image processing unit 420 performs a calculation such as image reconstruction using a nuclear magnetic resonance signal.

The body movement information processing unit 430 generates information in which the body movement of the subject and the imaging are associated with each other (body movement-related information), and displays the information on the video display device 70. As the body movement information included in the body movement-related information, for example, the body movement itself detected by the body movement detection device 60 or a result of analyzing an occurrence position or a degree of the body movement for the body movement detected by the body movement detection device can be included. The analysis of the body movement is performed by a body movement analysis unit 431 in the body movement information processing unit 430, and for example, in a case where the body movement detection device 60 is a camera, the position where the body movement occurs and the degree of the body movement are obtained using a video of the camera, and an allowable limit of body movement for each part or the like is analyzed.

The body movement information processing unit 430 (body movement-related information generation unit 432) also creates the body movement-related information to be presented to the subject based on the analysis result of the body movement analysis unit 431. For example, in a case where the degree of the body movement and the position where the body movement occurs are analyzed, the display content corresponding to an allowable limit of the body movement preset corresponding to the examination part is created as related information. The body movement-related information can include information for motivating the subject to keep a stationary state or information having entertainment properties.

The calculation unit 40 that performs the above-described processing can be configured with a general-purpose computer including a memory and a CPU, and the above-described processing is realized by the CPU reading and executing processing programmed in advance. However, a part of the processing performed by the calculation unit 40 may be performed by a programmable IC, such as an ASIC or an FPGA. A storage device 80 that stores data or the like required for the processing is connected to the calculation unit 40. The storage device 80 may be connected to the computer as a computer-dedicated storage device of the MRI apparatus, or may be a storage device (including the cloud and a portable medium) placed in a location different from the MRI apparatus.

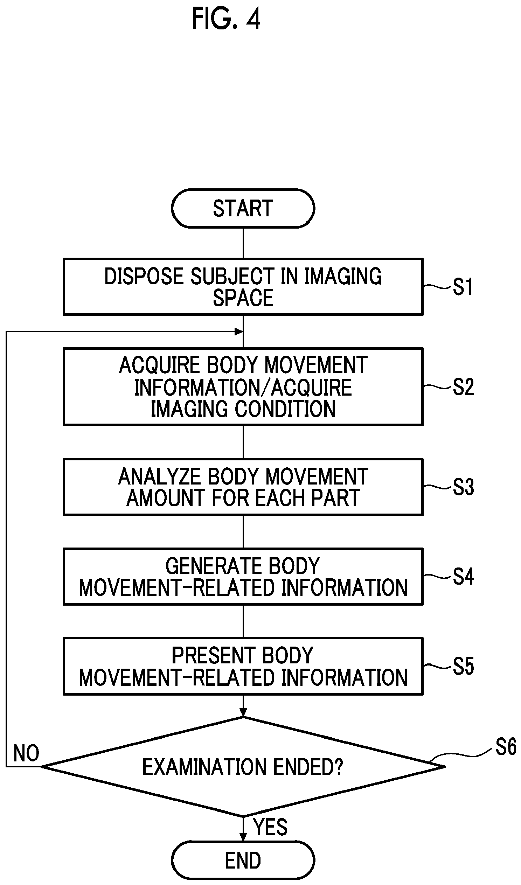

An outline of processing of the MRI apparatus having the above configuration will be described with reference to Fig. 4.

In a case where the examination is started and the subject is disposed at a predetermined position in the imaging space, the body movement detection device 60 starts to monitor the body movement of the subject before and after the disposition (S1 and S2). In a case where the body movement detection device 60 is a camera, the camera video may be used for the disposition of the subject, but the monitoring of the body movement targets the body movement of the subject after the disposition of the subject.

The body movement information (in a case where the body movement detection device 60 is a camera, a video thereof) detected by the body movement detection device 60 is passed to the body movement information processing unit 430. The body movement information processing unit 430 acquires information related to the imaging currently being performed (for example, information on an examination part, whether or not the imaging is robust against body movement, an imaging time, and a progress status) from the imaging control unit 410, creates display information (referred to as body movement-related information) in which the information related to the imaging and the body movement information are associated with each other, and passes the display information to the display control unit 440 (S4). The processing from the monitoring of the body movement to the creation of the body movement-related information is repeatedly executed until the examination is ended (S6), and the display control unit 440 displays the body movement-related information on the video display device together with the other accessory information as a video (S5).

With the MRI apparatus according to the present embodiment, the subject can sequentially obtain information indicating that the body movement of the subject himself/herself does not occur or occurs during the progress of the examination or information on a part or a time slot where a certain degree of body movement is allowed, together with information on the progress of the examination, through the video display device at the same time as the examination is started. As a result, it is possible to reduce a psychological burden of feeling stress, boredom, and anxiety due to not knowing the body movement that has no influence on the examination felt by a patient during the examination.

In addition, the examinee is not simply forced to be in a stationary state to receive the examination, but is motivated to actively maintain the stationary state, which leads to an improvement in the acceptance for the long-time examination and makes it easy to maintain the stationary state, thereby reducing the body movement.

Next, an embodiment of specific processing of the body movement information processing unit will be described.

### <Embodiment 1>

In the present embodiment, information on the examination part and information from the body movement detection device (camera) 60 are presented to the subject, and a message related to the body movement is displayed. The message is a message indicating how much the body movement can be allowed in relation to the examination part, a message indicating that the body movement occurs, or the like.

Fixed-form messages can be created in advance based on information on allowable movement of each part for each examination part, and the message related to the allowability of the body movement can be selected from the fixed-form message based on the information on the examination part acquired from the imaging control unit 410 and be displayed.

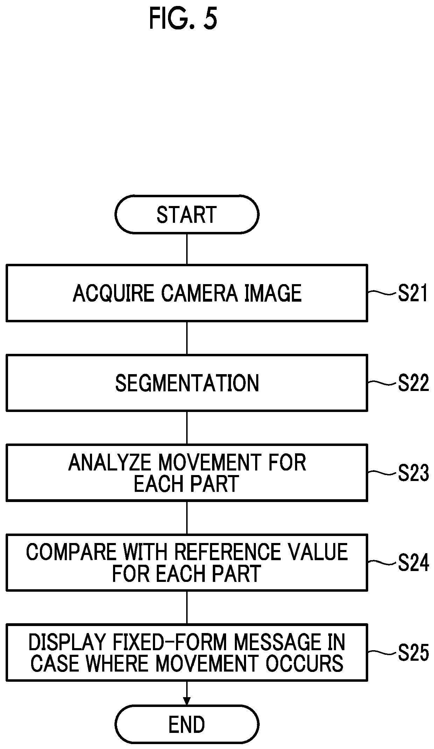

The determination of whether or not the body movement occurs is performed by the body movement analysis unit 431 analyzing the movement at each position based on the body movement information from the body movement detection device 60. Specifically, as shown in Fig. 5, in a case where the body movement information processing unit 430 receives the video of the subject from the camera (S21), the body movement analysis unit 431 analyzes the magnitude of the movement for each region of the video (image) (S22 to S24). A method of dividing the image into regions is not particularly limited, but segmentation is performed to divide the image into regions, for example, a head, a trunk, and limbs (S22). Alternatively, the image may be divided into rectangular regions separated by, for example, vertical and horizontal lines, and each rectangular region may be registered in advance in a part (head, trunk, or limb) to which the rectangular region belongs. The movement is analyzed by using a method such as an optical flow for a plurality of images obtained at predetermined time intervals to calculate the magnitude of the movement at that time (S23).

The body movement analysis unit 431 further compares the magnitude of the movement with a reference value (S24). As the reference value, a position at a timing of disposing the subject in the examination space (at a timing of start of examination) may be used, or an average value of changes in the body movement acquired within a predetermined time after disposing the subject in the examination space can be used. In a case where the magnitude is larger than the reference value, it is determined that there is unallowable movement. A fixed-form message is output based on the determination result (S25).

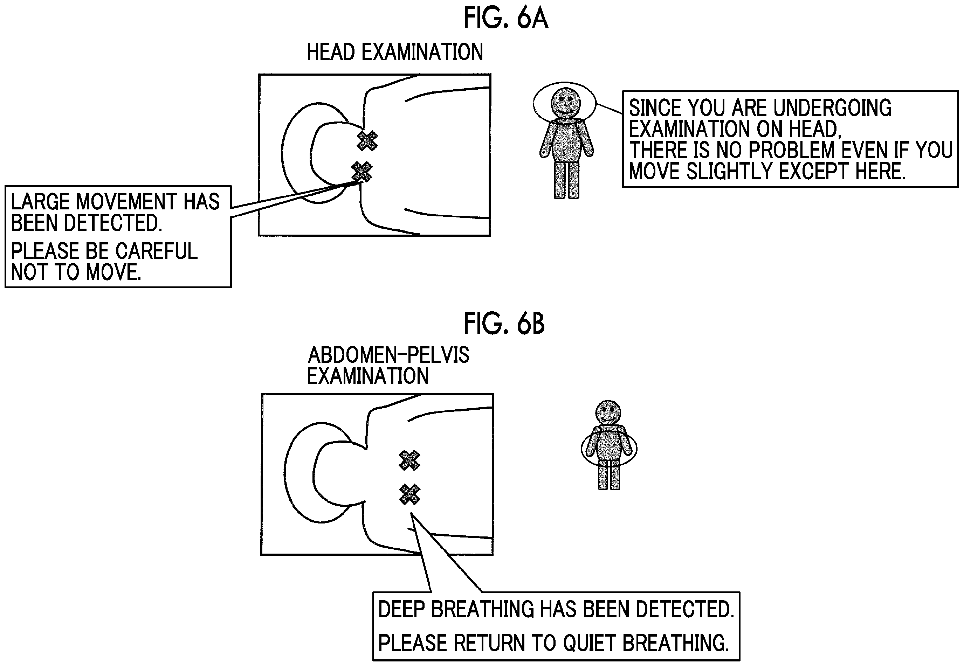

Figs. 6A and 6B show examples of the body movement-related information displayed on the video display device 70. The example shown in Fig. 6Ais a display example in a case of a head examination. First, a mark (here, a circle surrounding the examination part) indicating the examination part is shown on an illustration of a human body, and a message regarding an allowable degree of the body movement, for example, "Since you are undergoing an examination on a head, there is no problem even if you move slightly except here." is displayed. In addition, as the body movement information of the subject himself/herself, the video acquired by the camera is displayed. The body movement analysis unit 431 superimposes and displays, for example, a mark such as "X" on the video at a position in which it is determined that the movement occurs.

The example shown in Fig. 6B is a display example in a case of an abdomen-pelvis examination, and is the same as the example in Fig. 6A in that a mark indicating the examination part is shown on an illustration of a human body. But in this example, in the video display of the subject, a region-of-interest of the display is further extracted in accordance with the examination part or the part where the body movement is detected is further displayed in an enhanced manner. In addition, in a case where the body movement of a level at which the examination is influenced is detected, a message for reporting the detection, for example, "Deep breathing has been detected. Please return to quiet breathing." is displayed.

Further, a part where the movement is not always desired may be specified by an illustration or the like until the examination is ended, or a period in which the movement is not particularly desired, such as during the main scan imaging, may be specified by changing a color or a display method. In general, in the MRI examination, since the examination target part is often hidden by being covered with the reception coil, such a part is specified to enable the subject to be aware of the part.

In the examples shown in Figs. 6A and 6B, the information on the allowable movement in the examination part is displayed as text information together with the illustration, but the part of the movement that has influence may be displayed as a drawing pattern depending on the examination part.

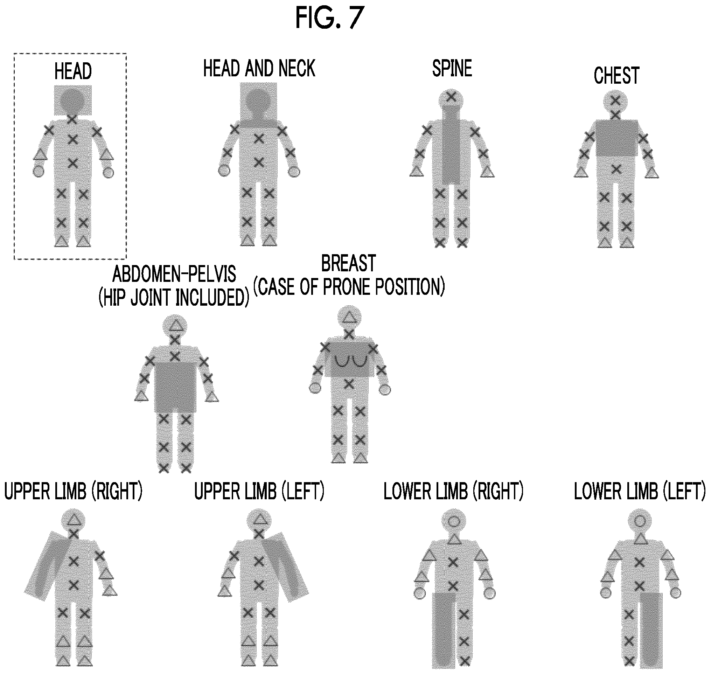

Fig. 7 shows an example of a drawing pattern showing a relationship between the examination part and the body movement. As shown in the drawing, this drawing pattern indicates an examination part with a square and attaches a mark indicating the allowable limit of the movement to other parts on an image in a shape of a human body. For example, in imaging of the head, the imaging of the head is indicated by a gray square, and marks "○" are attached to both hands with a highest allowable limit of the movement, marks "△" are attached to wrists and foots, and marks "×" are attached to the other parts. Similarly, the display of the parts being imaged and the display of a mark such as "○", "△", and "×" are performed for the other parts.

By displaying such a drawing pattern on the video display device 70 during the examination, the subject can be aware of his/her own examination part and a body movement suppression part to be noted and can make an effort to maintain the stationary state. It should be noted that, although performing only the display that presents the allowable limit of the movement as shown in Figs. 6A and 6B or Fig. 7 is also included in the present invention, by performing the display together with the warning message regarding actual movement, the subject can be aware of, for example, a part that the subject moved unconsciously or involuntarily, whereby it is possible to enhance the suppression of the movement.

According to the present embodiment, by presenting the information or a message regarding the allowable movement in the examination part and specifying a part where the movement has occurred or a part where the movement is not allowed to occur on a camera image obtained as the body movement information, it is possible to reduce a psychological burden of a subject, such as anxiety due to not knowing the body movement that has no influence. In addition, the probability of suppressing physiological movement or unconscious body movement increases, and the labor required for reimaging can be reduced.

### <Embodiment 2>

In the present embodiment, as the body movement-related information, a display in which the content of the warning issued for the movement occurring in each part of the subject is changed depending on whether the imaging sequence is robust with respect to the body movement is displayed.

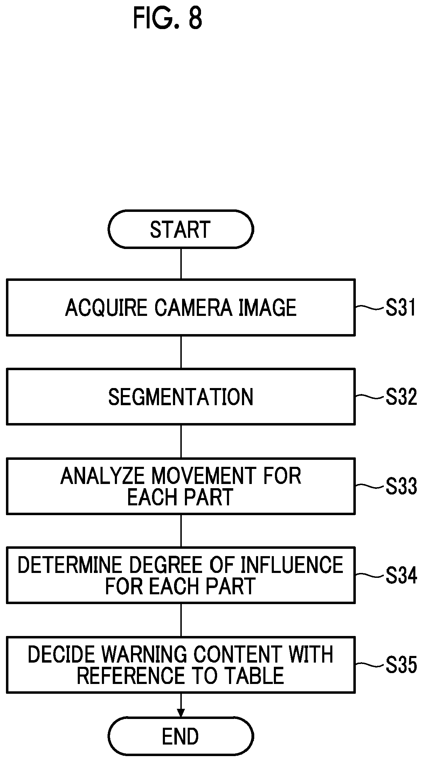

Hereinafter, an operation of the MRI apparatus according to the present embodiment will be described with reference to a flow of processing of the body movement information processing unit 430 shown in Fig. 8. Here, it is assumed that a camera that acquires a video of the subject in the imaging space is used as the body movement detection device 60.

In the body movement information processing unit 430, in a case where the video of the subject is transmitted from the camera (S31), the body movement analysis unit 431 analyzes the magnitude of the movement for each region of the video (image) (S32 and S33). The method of dividing the region of the image and analyzing the movement for each region is the same as the method described in Embodiment 1.

The body movement analysis unit 431 compares the magnitude of the movement with a preset threshold value to determine a degree of influence (S34). The influence of the magnitude of the movement on the imaging is different depending on the imaging sequence and the examination part. For example, in an SE system sequence, an FSE system sequence, or 3D imaging, the imaging time is long, and thus the imaging is likely to be influenced by the body movement. In addition, for example, in imaging targeting the head, the movement of the head leads to the degradation of the image quality as it is, but the movement of the limbs are less influenced. Therefore, the threshold value is preset according to the imaging sequence and the examination part, and the warning is different between a case of large movement exceeding the threshold value and a case of movement within the threshold value.

The body movement analysis unit 431 compares the magnitude of the movement for each analyzed part with a threshold value based on the imaging information indicating whether or not imaging using the sequence, in which imaging is easily affected by the body movement, is currently being performed and where the examination part is, and displays the necessary warning on the video display device 70 (S35). In the present embodiment, the movement and the warning content for each imaging sequence and each examination part are tabulated in advance and stored in the storage device 80. The body movement information processing unit 430 displays the appropriate warning content by referring to the analysis result of the body movement analysis unit 431 and the table.

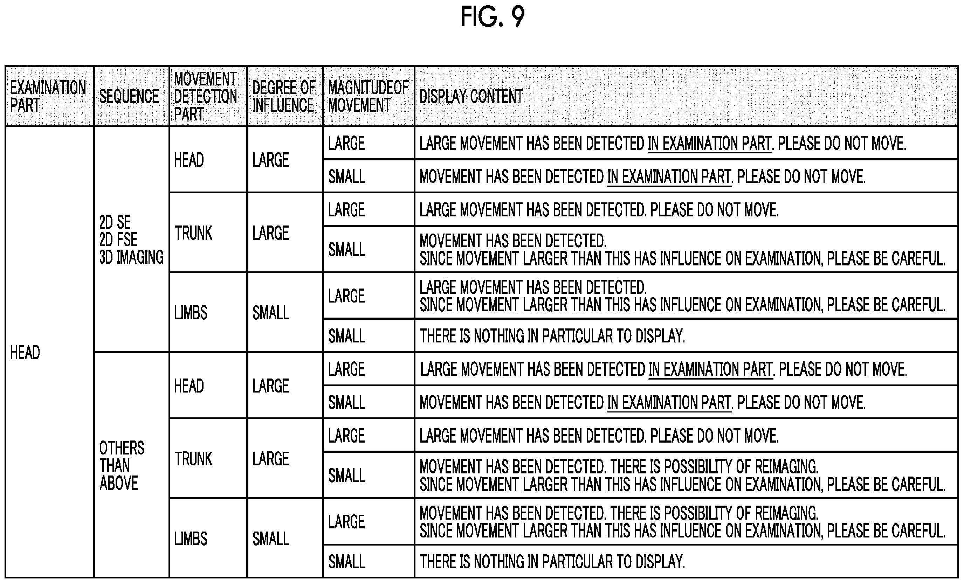

Fig. 9 shows an example of the table. In this table, the examination part is the head, and the warning content is assigned for each of the head, the trunk, and the limbs in accordance with the magnitude of the detected movement. In the imaging of the head using the SE system sequence, for example, in a case where it is determined as a result of the analysis of the body movement analysis unit 431 that the movement of the head is "large" (larger than the threshold value), a warning indicating that "Large movement is detected in the examination part. Do not move." is displayed on the video display device. Alternatively, in a case where the reimaging is required to be performed depending on the imaging even in a case where the movement occurs in the trunk or the limbs, a warning such as "There is a possibility of reimaging." is also displayed in accordance with the magnitude of the movement. The subject can be aware of his/her own movement through this display, and can be provided with an opportunity to make an effort to maintain the stationary state.

The table as shown in Fig. 9 can be prepared for each examination part, and the body movement information processing unit 430 appropriately selects and applies the table to be referred to depending on the examination part to display an appropriate warning.

In the above description, a case where the body movement detection device 60 is a camera has been described as an example, but for example, in imaging of an abdomen or the like, in a case where a body movement detector that detects the magnitude of the respiratory movement is provided as the body movement detection device 60, a cycle of the respiratory movement of the subject may identified from a detection position detected by the body movement detector before the imaging, the predetermined time phase, for example, the time phase of exhalation may be set to be a reference value, and the magnitude of the movement and disturbance of the body movement may be determined with the reference value as a threshold value. In that case, not only a warning according to the magnitude of the movement but also a warning corresponding to the disturbance of the body movement may be prepared and displayed.

In addition, not only the display of the warning but also the warning using voice or the like can be performed together, so that the effect of the warning can be obtained even for the subject with a visual impairment or the subject who is asleep. The same display content as the display content of the video display device for being viewed by the subject may be displayed on a monitor (not shown) for an operator, whereby the operator can confirm that an appropriate warning is issued.

According to the present embodiment, for the imaging in progress, the subject can autonomously and effectively suppress the movement particularly for the part where the movement is not allowed, while confirming his/her own movement.

### <Modification Example 1 of Embodiment 2>

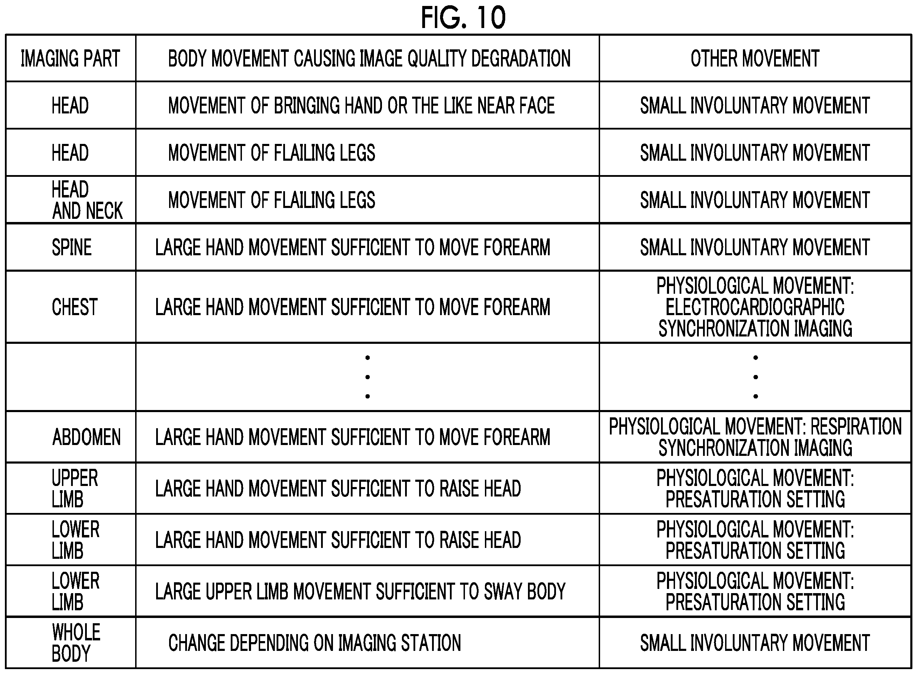

Fig. 9 is an example of a table referred to in a case where only the magnitude of the movement is analyzed, but the body movement analysis unit 431 can also analyze the characteristics of the movement (whether the movement is temporary and large movement or small and cyclical movement) or the cycle of the movement by analyzing the change in the movement over time, and may display a warning by taking into account the degree of influence on the imaging due to the characteristics of the movement. For example, as shown in Fig. 10, a warning may be issued with reference to Fig. 9 only in a case where it is determined that the characteristic of the movement, for which it is determined that there is a large influence on the imaging, is present in the table, by tabulating the characteristic of the movement and the degree of influence on the imaging for each examination part and referring to a result of the analysis on the characteristic of the movement performed by the body movement analysis unit 431 together with the table. Alternatively, the warning content according to the characteristic of the movement, which is different from the warning in Fig. 9, may be prepared in advance and issued.

According to the present modification example, it is possible to provide the subject with more accurate information regarding the body movement, and it is possible to increase a sense of relief of the subject.

### <Embodiment 3>

In Embodiments 1 and 2, the body movement during the imaging is the problem, but the present embodiment relates to the display of the body movement-related information in an examination in which imaging is continuously performed for a plurality of times.

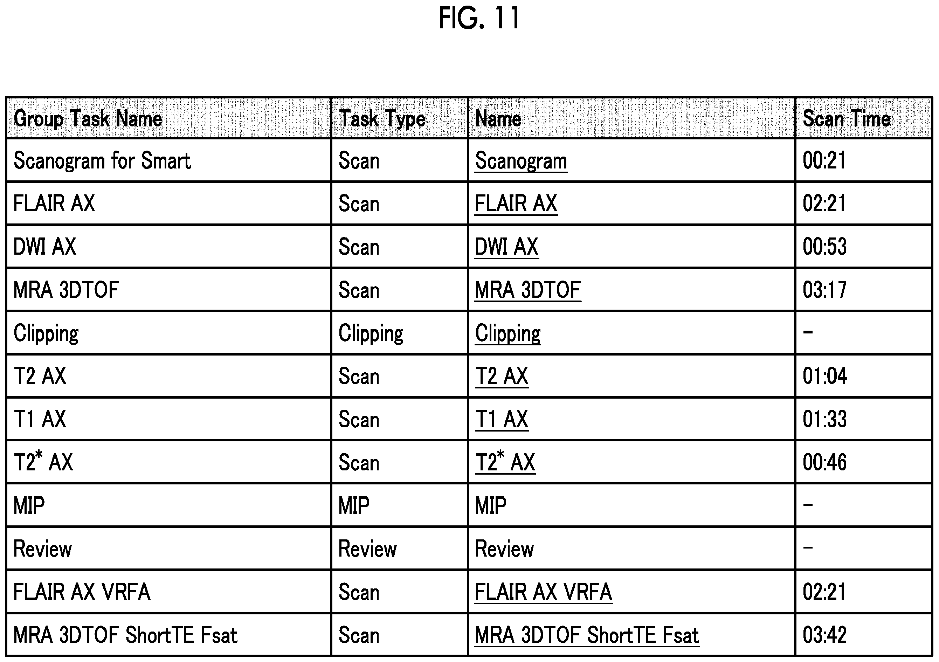

In the MRI examination, even in a case where one examination part is imaged, in many cases, first, imaging starts with the positioning imaging, and then imaging (main scan) using a plurality of types of imaging sequences having different contrasts is performed. In addition, in some cases, the image after imaging is checked, and additional imaging (additional scan) is performed. An examination flow of MRI consists of a series of the main scans. Fig. 11 shows an example of the MRI examination flow. The operator performs the examination based on such an examination flow, but this information is not shared with the subject who is being examined. In the present embodiment, a timing in which small movement is allowed or the like is presented to the subject by associating the body movement of the subject with the examination flow.

In principle, in the examination flow, it is desirable that the subject does not move after the positioning during a series of main scans regardless of the degree of influence of the movement, but during an interval between the main scan and the main scan or during image check, small movement in a part having a small degree of influence without a position change is allowed. However, the interval between the main scan and the main scan or the time during image check may take several tens of seconds to several minutes, or may take only several seconds.

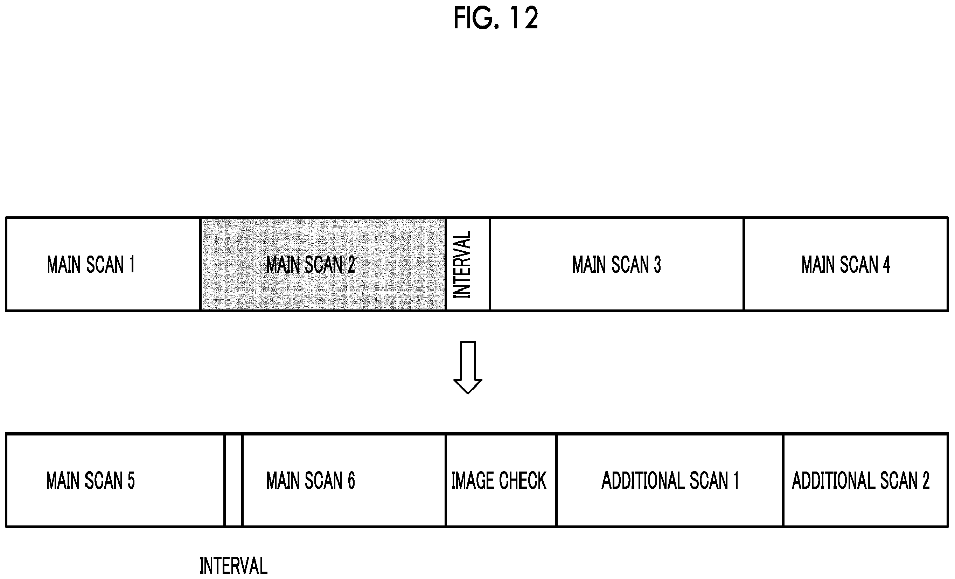

In the present embodiment, a progress state of the imaging is presented, and information on a time in which the subject is allowed to move is provided. A presentation method is not particularly limited, but for example, a table of the examination flow as shown in Fig. 11 is displayed, and a color display indicating that the main scan is in progress is performed. After one main scan ends, a color blinking display is performed for the next main scan until the next main scan starts to indicate the interval. In this case, a display for visualizing a time to the next main scan (for example, a figure display such as an hourglass) may be performed, the blinking display or the display for visualizing a time may be performed only in a case where the interval is longer than a predetermined time, and a color display indicating that the next main scan is in progress may be performed in a case where the interval is extremely short (about several seconds). Instead of the table, a timing chart showing the progress status as shown in Fig. 12 may be used.

In addition, although the same display as the display between the main scans may be performed even during the image check, a different display method, such as displaying a message (text display) for indicating that the check is being performed and instructing not to move as much as possible during the confirmation, may be adopted as a display during the image check.

The same display as the display in Embodiments 1 and 2 is performed during the main scan.

According to the present embodiment, the subject can make the movement, which has been suppressed, such as coughing or sneezing in the presented interval or image check, so that it is possible to relieve the pain of the subject. In addition, since the subject can check the progress status of the examination flow, the subject can know the prospect of the end of the examination and can receive the examination with relief.

### <Embodiment 4>

In the present embodiment, content in which information related to the body movement and the fact that the subject maintains the stationary state are evaluated is displayed. A degree of the progress of the examination may be further displayed. Various aspects can be considered for the evaluation content, but the evaluation content is set to content in which a drawing pattern displayed to encourage the subject being examined is changed in accordance with the time during which the stationary state is maintained. In addition, the drawing pattern is appropriately changed depending on whether the subject is a child or an adult.

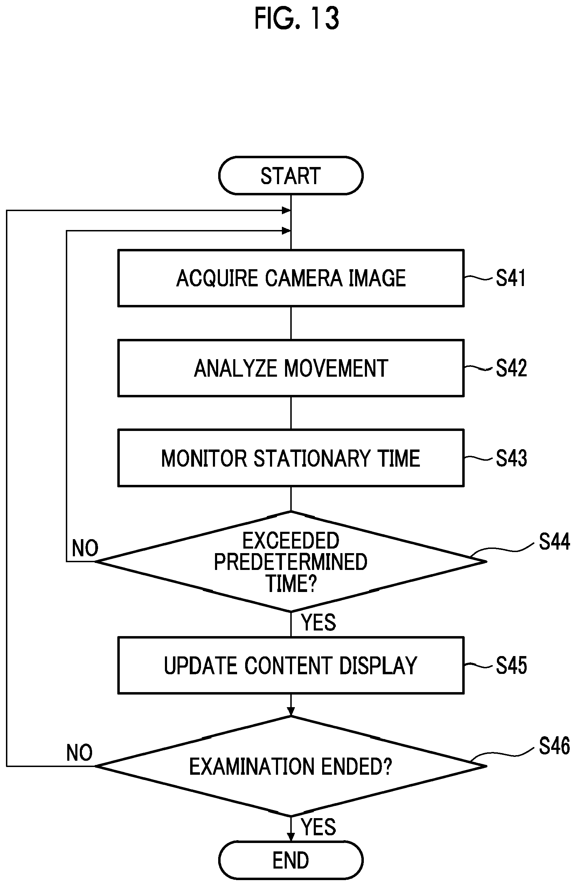

Fig. 13 shows a flow of processing of the body movement information processing unit 430 according to the present embodiment. Here, the acquisition of the camera image and the analysis of the movement (S41 and S42) are the same as the processing shown in Fig. 5 or 8. In the present embodiment, a time (stationary time) in which there is no movement is further monitored (S43), and in a case where the stationary time exceeds a predetermined time (S44), the displayed content is updated and changed to, for example, a drawing pattern in which a character has evolved. The drawing pattern is changed such that the evolution of the character progresses while the stationary time is continued (S41 to S45).

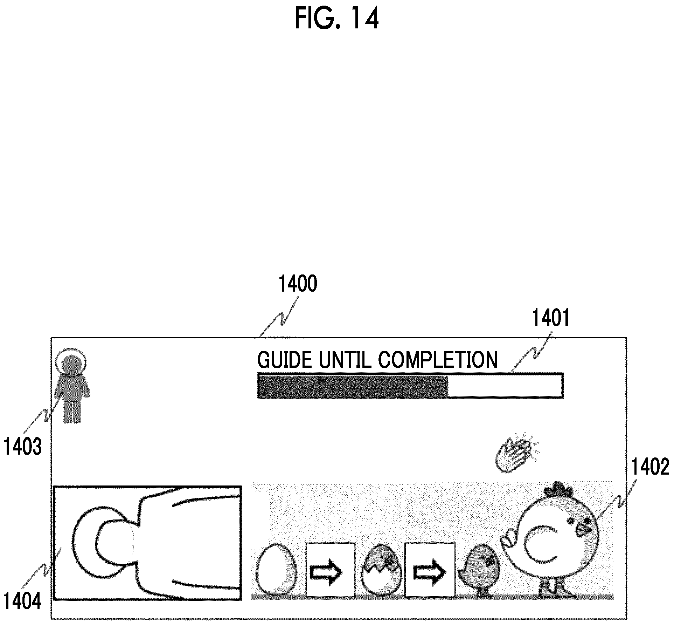

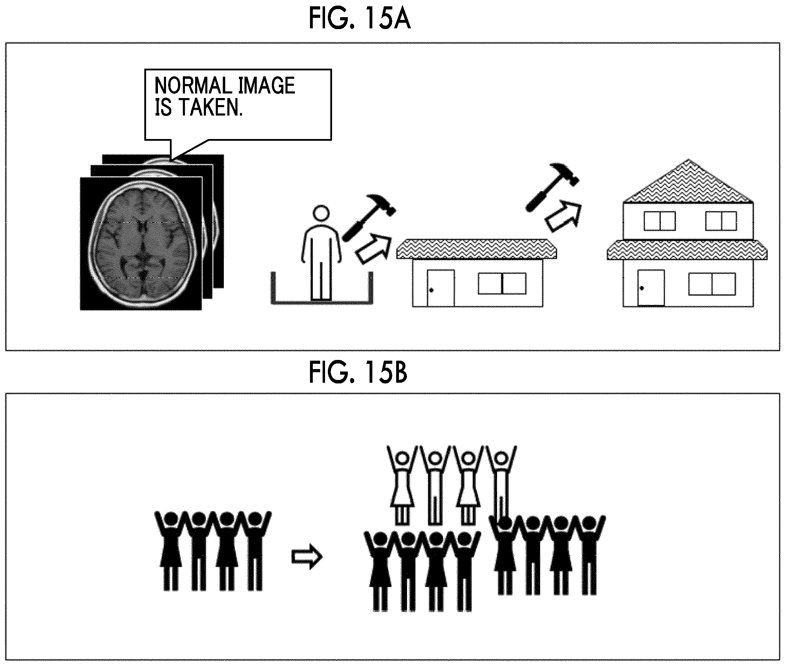

Examples of the content are shown in Figs. 14 and 15. Fig. 14 is an example of the content for children, and provides content having a gaming property, such as a character change, in order to advance the progress of the examination. On a screen 1400, a bar graph 1401 showing a progress state of the imaging and an evolving character 1402 are displayed as information indicating the relationship between the imaging and the body movement. Further, although not essential in the present embodiment, an icon (a figure imitating a person) 1403 indicating the examination part and a video 1404 of the subject captured by the body movement detection device 60 (camera) are displayed together with the information indicating the relationship between the imaging and the body movement.

The character 1402 is, for example, small at the start of imaging (for example, a leftmost image is displayed), but grows and changes to an evolved character (from left to right) in a case where the stationary state is maintained. By seeing such a change, the subject (child) enjoys the change itself, and feels the change as a reward for not moving, and is motivated to further maintain the stationary state. A plurality of characters may be prepared as the character 1402, and the subject may be allowed to select the character.

In addition, since a loud noise as in construction work, such as "clang clang" or "knock knock", is generated during the imaging of the MRI, the content that does not make a sense of incongruity to the sound may be provided. Fig. 15A is an example thereof, and displays a video in which a building under construction and a craftsman (carpenter) involved in the building are displayed instead of the character. Also in this case, the building approaches completion with the passage of time during which the stationary state is maintained. In addition, the drawing pattern of the craftsman appears in a case where the subject does not move, but disappears in a case where the subject moves. As a result, the subject becomes aware that the subject has moved, feels pleasure in the progress of the building construction due to the fact that the subject does not move, and is motivated to further maintain the stationary state. By sequentially displaying an MR image formed at the same time, the subject can feel the completion of the examination.

Fig. 15B is an example of the content of praising by increasing the number of happy families and medical staffs, in which the number of people around the subject who watch the subject and praise the subject making an effort to maintain the stationary state is increased in accordance with a continued time of the stationary state. Even in the examples shown in Figs. 15A and 15B, as in the screen example shown in Fig. 14, the camera image may be displayed, or as shown in Fig. 15A, the captured image may be displayed instead of the camera image or together with the camera image. In this case, by adding a message such as "A normal image is taken." to praise the subject, it is possible to further relax the subject or to increase motivation to maintain the stationary state.

Figs. 14 and 15 are merely examples of the display content, and various kinds of content may be used. The various kinds of content may be prepared, and content suitable for the patient may be selected and applied.

According to the present embodiment, in a boring MRI examination, the subject is requested to cooperate with the examination and is entertained with the content, so that the suppression of the body movement can be expected and the subject can approach the examination with a positive mindset. Since the age and the way of enjoying of the patient vary, it is possible to contribute to the reduction of the stress in which the patient is not forcibly relaxed, by preparing the content suitable for the patient.

### <Embodiment 5>

In general, in the abdominal examination, there are various imaging methods such as imaging that requires breath holding and respiratory synchronization imaging (or diaphragm-synchronization) that obtains an image by repeating a certain breathing, depending on the imaging sequence. In order to obtain an image correctly, it is necessary to have the subject hold his/her breath firmly or to have the subject continue to breathe regularly. In the present embodiment, in order to deal with imaging that requires breath holding or respiratory synchronization imaging, information on the movement of the subject is made to be seen not only by the operator but also by the patient himself/herself to make the operator and the patient understand a good or bad respiratory state.

In the breath-holding imaging or the synchronization imaging, a monitor for monitoring the respiratory movement of the subject is installed as the body movement detection device 60. The body movement information processing unit 430 according to the present embodiment acquires information from the body movement detection device (monitor), determines the respiratory movement of the subject and whether or not the respiratory movement of the subject is in an appropriate state for the purpose of imaging, and presents the determination result together with the body movement information. The determination of whether or not the respiratory movement is appropriate is performed by the body movement analysis unit 431 analyzing, for example, the magnitude of the movement and a duration time of the fluctuation in a certain magnitude of the movement based on the information from the body movement detection device 60 and determining whether or not the movement exceeding the predetermined threshold value continues for a predetermined duration time or whether or not the stationary state in which the magnitude of the movement is equal to or smaller than the threshold value continues for the predetermined duration time. The threshold value may be used as a level set by the operator based on the respiratory waveform of the subject measured in advance, or may be automatically set on the apparatus side.

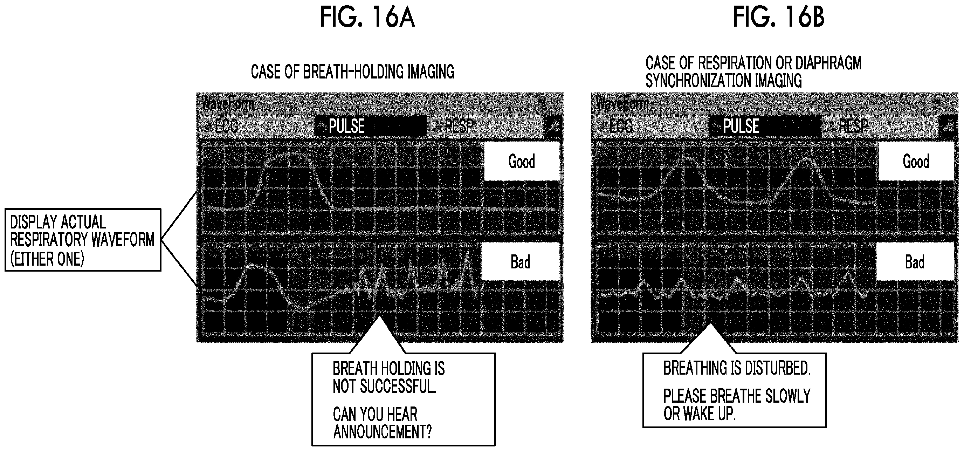

The present embodiment will be described with reference to a specific example of display content shown in Figs. 16A and 16B.

Fig. 16A is an example of the display content (body movement-related information) in a case of the breath-holding imaging, and in this example, the respiratory waveform detected by the body movement detection device is displayed as the body movement information. A case where breath holding is well performed is shown on an upper side, and a case where breath holding is not well performed is shown on a lower side, which has influence on the image quality. In Figs. 16A and 16B, two cases are shown in parallel, but actually, the current respiratory waveform of the subject, that is, one of the respiratory waveforms is displayed. In a case where the breath holding is successful or unsuccessful, the evaluation indicating the success or failure is displayed together.

As described above, the determination of the evaluation is made based on the magnitude of the movement and the duration time, and an evaluation, such as "Good" in a case where it is determined that the breath holding is successful based on the respiratory waveform or "BAD" in a case where the movement that has influence on the image quality occurs, is displayed. In a case where the breath holding is not successful, the subject is usually notified of the fact by an announcement, but the subject may not hear or understand the announcement in the imaging space. Therefore, in addition to the above-described evaluation, for example, a message such as "The breath holding is not successful." or "Can you hear the announcement?" is displayed.

In Fig. 16B, as in Fig. 16A, as a case of respiration or diaphragm synchronization imaging, a case where the synchronization imaging is well performed with a stable respiratory waveform is displayed on an upper side, and a case where the synchronization imaging is difficult with an unstable respiratory movement is displayed on a lower side, while a waveform of the actual subject (that is, one of the waveforms) is displayed. In this case, the body movement analysis unit 431 may analyze a cycle of the fluctuation in addition to the magnitude of the movement, and in that case, the movement may be evaluated based on the stability of the cycle. In a case where the respiratory movement is unstable as in the lower side, for example, a message such as "Breathing is disturbed. Please breathe slowly or wake up." is displayed in addition to an evaluation "BAD".

In addition to the displays shown in Figs. 16A and 16B, the display content shown in other embodiments, for example, the guide until completion as shown in Fig. 12 or Fig. 14 or the image that changes in accordance with the stationary duration time as shown in Figs. 15A and 15B may be displayed.

According to the present embodiment, it is possible to prevent reimaging and a delay in an examination time by notifying the subject of the abnormal respiratory state. In addition, it is possible to reduce the psychological burden of feeling stress, boredom, and anxiety due to not knowing the body movement that has no influence on the examination felt by a patient during the examination. Further, according to the present embodiment, since the determination of the movement in the breath-holding imaging or the synchronization imaging is automated, it is possible to reduce the variation in the movement determination for each operator.

Above, the flow of the processing of the body movement information processing unit of the MRI apparatus according to the embodiment of the present invention and the embodiments of the body movement-related information presented to the subject have been described. However, the present invention is to present, to the subject, information (message, image, video, and the like) on the body movement that is allowed or not allowed in relation to the examination or imaging in the examination that the subject is actually receiving, in association with the imaging, and various changes can be made to the method of determining the body movement and the display form of the presented content, and such modification examples are also included in the present invention. Further, the content described in each embodiment may be appropriately combined or some components may be omitted as long as there is no technical contradiction, and such modification examples are also included in the present invention.

Further, although the MRI apparatus has been described as an example of the medical image diagnosis apparatus, the present invention is also applied to a medical image diagnosis apparatus other than the MRI apparatus, such as a CT apparatus, as long as the medical image diagnosis apparatus captures an image with the subject held in a predetermined examination space.

For example, although not shown, the CT apparatus comprises a scanner mounted with an X-ray source and an X-ray detector as an imaging unit, in which the X-ray detector detects transmission X-rays emitted from the X-ray source and transmitted through a subject, and collects transmission X-ray data at each angle while rotating the scanner. In addition, the CT apparatus comprises a calculation unit that generates a tomographic image of the subject using the collected transmission X-ray data. In such a CT apparatus, the subject is inserted into a gantry (inside an opening of the scanner) that houses the scanner in a state of being placed on a bed, and a CT examination is performed.

Configurations of the imaging units accommodated in the gantries are different, but the CT apparatus also has an appearance similar to the appearance of the MRI apparatus shown in Fig. 1, and as shown in Fig. 1, the video display device 70 and the body movement detection device 60 are installed. As a function in the calculation unit, the body movement information processing unit 430 shown in Fig. 3 is provided. The functions of the body movement information processing unit 430 are the same as those of the MRI apparatus, and for example, the same processing as that in Figs. 4 and 13 is performed. The body movement-related information and the content displayed on the video display device 70 are the same as those of the MRI apparatus, and the same effects can be obtained, that is, the subject can autonomously suppress the body movement, and the subject can enjoy the content or be encouraged without getting bored or feeling anxiety due to the long examination time.

### Explanation of References

1: MRI apparatus
10: gantry
20: imaging unit
30: table
40: calculation unit
50: subject
60: body movement detection device
70: video display device
80: storage device
410: imaging control unit
430: body movement information processing unit
431: body movement analysis unit
432: body movement-related information generation unit
440: display control unit

## Claims

1. A magnetic resonance imaging apparatus comprising:
an imaging unit configured to acquire a magnetic resonance signal of a subject and generate an image of the subject; and
a body movement information processing unit configured to display body movement-related information, in which body movement information from a body movement detection device that detects body movement of the subject disposed in an imaging space and imaging information of the imaging unit are associated with each other, at a position where the subject disposed in the imaging space is capable of visually recognizing the body movement-related information, using a video display device.

2. The magnetic resonance imaging apparatus according to claim 1,
wherein the imaging information includes an examination part, and
the body movement-related information includes information in which the examination part and body movement information of a part of the subject capable of being detected by the body movement detection device are associated with each other.

3. The magnetic resonance imaging apparatus according to claim 1 or 2,
wherein the body movement-related information includes allowance of the body movement or a warning based on the body movement detected by the body movement detection device.

4. The magnetic resonance imaging apparatus according to one of the preceding claims,
wherein the body movement information processing unit
includes a body movement analysis unit configured to analyze the body movement detected by the body movement detection device, and
to display display content corresponding to an allowable limit of body movement preset corresponding to an examination part as the body movement-related information, based on an analysis result of the body movement analysis unit.

5. The magnetic resonance imaging apparatus according to claim 4,
wherein the body movement analysis unit is configured to analyze an allowable limit of body movement for each part, based on a relationship between the examination part and a part where the body movement occurs.

6. The magnetic resonance imaging apparatus according to claim 4 or 5,
wherein the body movement detection device includes a camera that images the subject, and
the body movement analysis unit is configured to analyze a degree of the body movement and a position or a part where the body movement occurs using a video of the camera.

7. The magnetic resonance imaging apparatus according to one of claims 4 - 6, further comprising:
a storage unit that stores a message corresponding to a degree of body movement at a part where the body movement occurs and an allowable limit of the body movement for each examination part as a table,
wherein the body movement information processing unit is configured to select the message based on the analysis result of the body movement analysis unit with reference to the table.

8. The magnetic resonance imaging apparatus according to claim 7,
wherein the imaging information includes a type of an imaging sequence, and
the allowable limit of the body movement included in the table is set in accordance with the imaging sequence.

9. The magnetic resonance imaging apparatus according to one of the preceding claims,
wherein the body movement-related information includes prior information indicating an allowable limit of body movement for each part corresponding to an examination part.

10. The magnetic resonance imaging apparatus according to one of the preceding claims,
wherein the imaging unit is configured to execute a series of imaging including a plurality of main scans, and
the body movement-related information includes timing information for allowing body movement in the series of imaging.

11. The magnetic resonance imaging apparatus according to one of the preceding claims,
wherein the body movement-related information includes an evaluation of a stationary duration time of the subject.

12. The magnetic resonance imaging apparatus according to claim 11,
wherein the body movement-related information includes a drawing pattern or image information that is changed in accordance with the stationary duration time of the subject.

13. The magnetic resonance imaging apparatus according to one of the preceding claims,
wherein the body movement information processing unit is configured to display progress information of imaging on the video display device together with the body movement-related information.

14. The magnetic resonance imaging apparatus according to one of the preceding claims,
wherein imaging performed by the imaging unit is breath-holding imaging or respiratory synchronization imaging, and
the body movement information processing unit is configured to display an image indicating a cycle of respiratory movement detected by the body movement detection device and an evaluation of the image, as the body movement-related information.

15. A body movement information presentation method of presenting, to a subject disposed in an imaging space and being examined, body movement information of the subject himself/herself, the method comprising:
analyzing a magnitude of body movement of the subject for each part and determining whether or not the body movement is allowed based on a relationship between an examination part and a part where the body movement is detected; and
presenting a message in accordance with a determination result.

16. A body movement information presentation method of presenting, to a subject disposed in an imaging space and being examined, body movement information of the subject himself/herself, the method comprising:
monitoring body movement of the subject and a duration time of a stationary state; and
displaying a video that changes in accordance with the duration time of the stationary state.

17. A medical image diagnosis apparatus comprising:
an imaging unit configured to acquire an image of a subject; and
a body movement information processing unit configured to process body movement of the subject being imaged,
wherein the body movement information processing unit displays body movement-related information, in which body movement information from a body movement detection device that detects the body movement of the subject disposed in an imaging space and imaging information of the imaging unit are associated with each other, at a position where the subject disposed in the imaging space is capable of visually recognizing the body movement-related information, using a video display device.
